# EUROPEAN PATENT APPLICATION

(11) **EP 4 682 238 A1**
(43) Date of publication of application: **21.01.2026**
(21) Application number: 23928532.3
(22) Date of filing: 17.03.2023
(51) Int. Cl.: C12M 1/04, C12N 1/00, C12N 1/20

(54) **MICROORGANISM CULTURING SYSTEM AND METHOD FOR CULTURING IN SAID SYSTEM BY RECYCLING OFF-GAS**

(71) Applicant: JGC HOLDINGS CORPORATION, Yokohama-shi, Kanagawa 220-6001 (JP)
(72) Inventor: OHBUCHI, Takayuki, Yokohama-shi, Kanagawa 220-6001 (JP); WATANABE, Yoshiyuki, Yokohama-shi, Kanagawa 220-6001 (JP); UENO, Yoshiki, Yokohama-shi, Kanagawa 220-6001 (JP); OKINO, Shohei, Yokohama-shi, Kanagawa 220-6001 (JP); MIZUGUCHI, Yoshihiro, Yokohama-shi, Kanagawa 220-6001 (JP)
(74) Representative: Becker, Eberhard
(86) International application number: PCT/JP2023/010685
(87) International publication number: WO 2024/194947

(57) **Abstract**

The present invention provides a microorganism culturing system, the culturing system comprising a culturing tank, a recycled gas adjustment unit, a first gas circulation line having one end connected to an upper part of the culturing tank and the other end connected to the recycled gas adjustment unit, and a second gas circulation line having one end connected to the recycled gas adjustment unit and the other end connected so as to supply the recycled gas into the culturing tank, wherein the recycled gas adjustment unit mixes one or more gas components selected from the group consisting of combustible gas, combustion-supporting gas, and incombustible gas with at least a portion of off-gas discharged from the culturing tank so that the concentration of the combustion-supporting gas in the recycled gas falls within a predetermined range. The present invention also provides a method for culturing in a culturing system that uses microorganisms by recycling off-gas.

## Description

### Technical Field

The present invention relates to a system for culturing a microorganism, and a method for culturing a microorganism, with an off-gas recycled, in the system.

### Background Art

Microorganisms can, due to the fermentation effect thereof, convert a gas containing much carbon, such as carbon dioxide, carbon monoxide, and methane, into various chemical products such as a fuel, a protein, an alcohol, and an organic acid, and are being industrially used. For the gas fermentation, various raw materials such as household waste, industrial waste, and agricultural waste can be used, and the gas fermentation can reduce the degree of dependence on carbon sources derived from fossils and contribute to the reduction of the discharge of greenhouse effect gasses, and is one of techniques drawing attention to realize sustainable society.

Microorganisms proliferate according to various conditions (for example, temperature, pH, pressure, stirring, and aeration) in a fermentation tank. Some microorganisms proliferate under anaerobic conditions, and other microorganisms proliferate under aerobic conditions. For the microorganism that proliferate under aerobic conditions, air is generally used as an oxygen source, but oxygen-enriched air and pure oxygen can also be used for the proliferation.

In order to optimize the productivity of products by a fermentation process using microorganisms that proliferate under aerobic conditions, culture at as high an oxygen concentration as possible is generally desired. Thereby, the amount of oxygen transferred from a gas phase and dissolved in a liquid phase is increased, and biosynthetic reactions of the aerobic microorganisms are increased.

Some types of microorganisms used for fermentation need a combustible gas (for example, a hydrogen gas) for a substrate of the fermentation thereof. Since the combustible gas may possibly cause gas explosion with a nearby ignition source, a culture process needs to be performed so as not to cause explosion. However, it is difficult to completely eliminate ignition sources such as static electricity, and therefore, the gas composition is generally controlled to be outside the range of explosive composition, that is, controlled so that the concentration of oxygen in the gas composition is not included in the explosion limit oxygen concentration.

A culture system is being developed that prevents gas explosion in a microorganism fermentation culture process using a combustible gas and contributes to the improvement of productivity of end products and to the reduction of operation costs. For example, Patent Literature 1 and Non Patent Literature 1 disclose a system for performing efficient culture by measuring the gas composition of an off-gas accumulated in a headspace of a fermenter after culture, and resupplying the off-gas to the fermenter simultaneously with mixing and supplying to the fermenter a component (for example, a hydrogen gas, an oxygen gas, and a carbon dioxide gas) in the gas composition consumed by fermentation.

### Citation List

### Patent Literature

Patent Literature 1: WO 2019/191767 A

### Non Patent Literature

Non Patent Literature 1: Garcia-Gonzalez, L., et al., Sustainable autotrophic production of polyhydroxybutyrate (PHB) from CO2 using a two-stage cultivation system., Catalysis Today, 257 (2015), pp.237-245

### Summary of Invention

### Technical Problem

An object is to provide a new system for culturing a microorganism, the system enabling safe and efficient culture, and a method for culturing a microorganism.

### Solution to Problem

In conventional systems (for example, Non Patent Literature 1) for culturing a microorganism, a method is employed in which when an off-gas accumulated in a headspace of a culture tank is reused, gas components insufficient for culture are separately premixed as a make-up gas and supplied to the culture tank. However, when the systems are used and a combustible gas is used, the possibility that the composition of the make-up gas supplied to the culture tank falls within the range of explosive composition cannot be denied. Therefore, a study has been conducted for a new system for culturing a microorganism, the system enabling safer and more efficient fermentation culture, and a method for culturing a microorganism.

As a result of conducting an earnest study to solve the problem, a system and a method have been developed that enable safe and efficient fermentation culture by mixing, for adjusting a recycled gas from an off-gas in a system for culturing a microorganism, desired gas components so that the concentration of a combustion-supporting gas in the recycled gas is in a prescribed range. That is, the present invention encompasses the following inventions.

[1] A system for culturing a microorganism, the system including:
   a culture tank;
   a recycled gas adjusting unit;
   a first gas circulation line having one end thereof connected to an upper part of the culture tank and another end thereof connected to the recycled gas adjusting unit; and
   a second gas circulation line having one end thereof connected to the recycled gas adjusting unit and another end thereof connected so as to supply a recycled gas into the culture tank,
   the recycled gas adjusting unit being connected to one or a plurality of gas feeders selected from the group consisting of a combustible gas feeder, a combustion-supporting gas feeder, and an incombustible gas feeder, and the recycled gas adjusting unit mixing, in at least a portion of an off-gas discharged from the culture tank, one or a plurality of gas components selected from the group consisting of a combustible gas, a combustion-supporting gas, and an incombustible gas so that a concentration of the combustion-supporting gas in the recycled gas is in a prescribed range.
[2] The system according to item 1, further including, in the second gas circulation line, a first gas concentration meter for measuring at least the concentration of the combustion-supporting gas in the recycled gas, wherein
   the recycled gas adjusting unit performs feedback adjustment, on the basis of the concentration of the combustion-supporting gas measured by the first gas concentration meter, so that the concentration of the combustion-supporting gas in the recycled gas falls within the prescribed range.
[3] The system according to item 1 or 2, wherein the combustible gas feeder is independently connected to the recycled gas adjusting unit.
[4] The system according to any one of items 1 to 3, wherein the recycled gas adjusting unit mixes the gas components so that a concentration of the combustible gas and/or the incombustible gas in the recycled gas is in a prescribed range.
[5] The system according to any one of items 1 to 4, further including, in the upper part of the culture tank and/or the first gas circulation line, a second gas concentration meter for measuring at least the concentration of the combustion-supporting gas in the off-gas.
[6] The system according to any one of items 1 to 5, wherein the combustion-supporting gas feeder and the incombustible gas feeder are connected to the recycled gas adjusting unit such that the combustion-supporting gas and the incombustible gas are premixed before mixed in the off-gas.
[7] The system according to any one of items 1 to 6, wherein the microorganism is an aerobic fermentation microorganism.
[8] The system according to item 7, wherein the aerobic fermentation microorganism is a hydrogen-oxidizing bacterium, and the combustible gas is a hydrogen gas.
[9] The system according to any one of items 1 to 8, wherein the combustion-supporting gas is an oxygen gas.
[10] The system according to any one of items 1 to 9, wherein the incombustible gas is a carbon dioxide gas and/or a nitrogen gas.
[11] The system according to any one of items 1 to 10, wherein a pressure in the culture tank is an atmospheric pressure or higher.
[12] The system according to any one of items 1 to 11, including a compressor in the first gas circulation line.
[13] The system according to any one of items 1 to 12, wherein the recycled gas adjusting unit includes:
   one or a plurality of gas mixers;
   a first gas adjusting unit for adjusting an amount of the combustible gas supplied from the combustible gas feeder;
   a second gas adjusting unit for adjusting an amount of the combustion-supporting gas supplied from the combustion-supporting gas feeder; and
   a third gas adjusting unit for adjusting an amount of the incombustible gas supplied from the incombustible gas feeder, and
   the combustible gas feeder, the combustion-supporting gas feeder, and the incombustible gas feeder are connected to the gas mixers.
[14] A method for culturing a microorganism, with an off-gas recycled, in a culture system using a microorganism, the method including:
   a step (1) of culturing a microorganism by supplying a supply gas containing a combustible gas, a combustion-supporting gas, and an incombustible gas to a culture material that is provided in a culture tank of a culture system and includes the microorganism and a medium;
   a step (2) of preparing a recycled gas by mixing, in at least a portion of an off-gas discharged by the step (1), one or a plurality of gas components selected from the group consisting of the combustible gas, the combustion-supporting gas, and the incombustible gas, the mixing being performed so that a concentration of the combustion-supporting gas in the recycled gas is in a prescribed range; and
   a step (3) of performing the step (1) by supplying, as the supply gas, the recycled gas obtained in the step (2) to the culture material.
[15] The method according to item 14, the method including, after the step (2) but before the step (3),
   a step (2') of measuring at least the concentration of the combustion-supporting gas in the recycled gas, wherein feedback adjustment is performed, on the basis of the concentration of the combustion-supporting gas measured in the step (2'), so that the concentration of the combustion-supporting gas in the step (2) falls within the prescribed range.
[16] The method according to item 14 or 15, wherein the combustible gas is independently mixed in the off-gas.
[17] The method according to any one of items 14 to 16, wherein the combustible gas and/or the incombustible gas is mixed in the gas components so as to be in a prescribed concentration range in the recycled gas.
[18] The method according to any one of items 14 to 17, including, after the step (1) but before the step (2),
   a step (1') of measuring at least the concentration of the combustion-supporting gas in the off-gas discharged by the step (1).
[19] The method according to any one of items 14 to 18, wherein in the step (2), the combustion-supporting gas and the incombustible gas are premixed before mixed in the off-gas.
[20] The method according to any one of items 14 to 19, wherein the microorganism is an aerobic fermentation microorganism.
[21] The method according to item 20, wherein the aerobic fermentation microorganism is a hydrogen-oxidizing bacterium, and the combustible gas is a hydrogen gas.
[22] The method according to any one of items 14 to 21, wherein the combustion-supporting gas is an oxygen gas.
[23] The method according to any one of items 14 to 22, wherein the incombustible gas is a carbon dioxide gas and/or a nitrogen gas.
[24] The method according to any one of items 14 to 23, wherein a pressure in the culture tank is an atmospheric pressure or higher.

### Advantageous Effects of Invention

According to the present invention, it is possible to provide a culture system and a culture method that have high safety, having a lower explosive risk than conventional methods when adjusting a recycled gas, reusing an off-gas, in fermentation culture of microorganisms.

### Brief Description of Drawings

FIG. 1 is a schematic diagram that illustrates one example of the configuration of a culture system according to the present invention.
FIG. 2 is a schematic diagram that illustrates one example of the configuration of the culture system according to the present invention.
FIG. 3 is a schematic diagram that illustrates one example of the configuration of the culture system according to the present invention.

### Description of Embodiments

Hereinafter, embodiments of the present invention are described. However, the technical range of the present invention is not limited only to the following embodiments. In the meantime, the prior art documents cited in the present description is herein incorporated by reference. The present invention can be modified, for example, by adding, removing, or replacing a constitutional requirement of the present invention without departing from the intent of the present invention.

In the present description, the terms "first", "second", "third", etc. are used to distinguish one element from another element. For example, a first element may be expressed as a second element, and similarly the second element may be expressed as the first element. Such expression does not depart from the range of the present invention.

Unless otherwise defined, the terms (technical terms and scientific terms) used in the present description have the identical meanings with the terms that a person skilled in the art generally understands.

### <System for culturing microorganism>

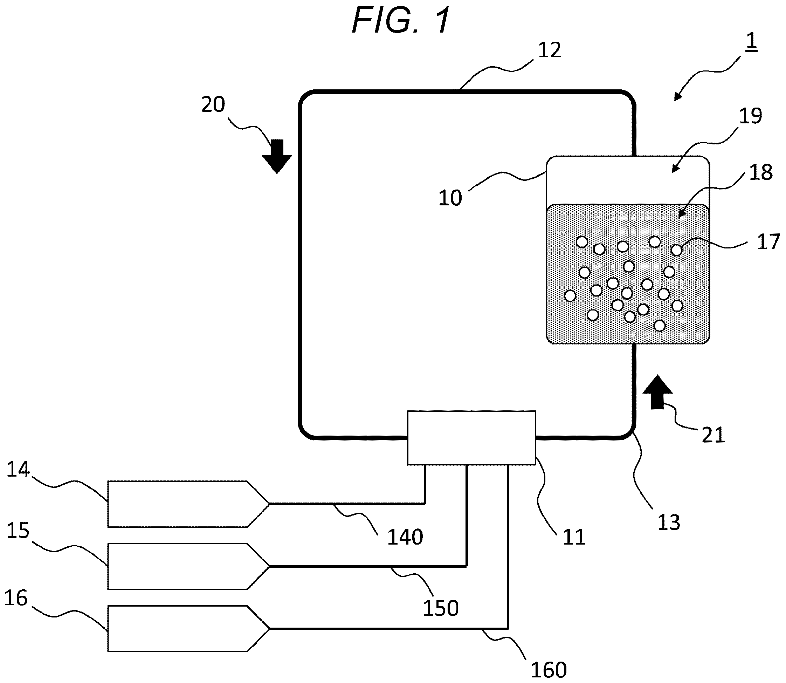

FIGs. 1 to 3 are schematic views that each illustrate a system (1, 1a, 1b), according to one embodiment, for culturing a microorganism. However, the configurations illustrated in the drawings of the present application are only examples, and the configuration of the culture system according to the present invention is not limited to these examples. In the present description, the elements having the identical reference sign assigned thereto are basically meant to indicate the identical elements. The elements configuring the culture system 1, 1a, or 1b may be used by employing only a part of the elements or by any combination of the elements according to the purpose or the necessity. The description about the elements of the culture system according to the present invention also applies as appropriate to a method according to the present invention described later.

In one aspect, a culture system 1 (for example, FIG. 1) according to the present invention includes:
a culture tank 10;
a recycled gas adjusting unit;
a first gas circulation line 12 having one end thereof connected to an upper part of the culture tank and another end thereof connected to the recycled gas adjusting unit; and
a second gas circulation line 13 having one end thereof connected to the recycled gas adjusting unit and another end thereof connected so as to supply a recycled gas into the culture tank.

In the culture system 1 according to the present invention, the recycled gas adjusting unit (for example, a gas mixer 11) is connected to one or a plurality of gas feeders selected from the group consisting of a combustible gas feeder 14, a combustion-supporting gas feeder 15, and an incombustible gas feeder 16, and the recycled gas adjusting unit is configured to mix, in at least a portion of an off-gas discharged from the culture tank 10, one or a plurality of gas components selected from the group consisting of a combustible gas, a combustion-supporting gas, and an incombustible gas (also referred to as an "inert gas") so that a concentration of the combustion-supporting gas in the recycled gas is in a prescribed range.

As the culture tank 10 employed in the culture system 1 according to the present invention, a culture tank can be used that is suitable for the type of fine matter to be cultured and the achievement of a purpose (for example, optimal conditions for producing a desired substance produced by the microorganism). The capacity and the shape of the culture tank 10 may be selected according to the use, the purpose, or the like, and are not limited. However, for example, a culture tank having a cylindrical shape, a bale shape, a spherical shape, or the like can be employed. In the culture tank 10, a stirring blade for stirring a medium may be set. The culture tank may stir a medium by the liquid flow generated through circulation of the medium. The culture tank 10 may stir a medium by shaking of the culture tank itself. The culture tank 10 may include a temperature controlling device (for example, a heater or a cooling coil) to keep the temperature of the medium at any temperature. In the culture tank 10, for example, a pH sensor, a dissolved oxygen (DO) sensor, and/or a pressure sensor for monitoring the conditions of the medium in the culture tank 10 may also be set. Devices and sensors set in known culture tanks can be employed by forming a combination thereof according to the use and the purpose.

The culture system 1 according to the present invention can be applied particularly to fermentation culture using a combustible gas. Example of the combustible gas applicable in the culture system 1 according to the present invention (and the method according to the present invention) include, but are not limited to, gasses such as hydrogen, ammonia, and hydrogen sulfide, and gasses obtained through volatilization of volatile hydrocarbons (examples thereof include, but are not limited to, methane and hydrocarbons having 2 or more carbon atoms (C) (for example, a C₂₋₈ alkane, a C₃₋₈ cycloalkane, a C₂₋₈ alkene, a C₃₋₈ cycloalkene, a C₂₋₈ alkyne, and benzene). For example, when the microorganism used for fermentation is a hydrogen-oxidizing bacterium, a hydrogen gas can be applied.

In order to prevent gas explosion of a gas mixture containing the combustible gas, it is crucial mainly (i) not to give an ignition source and (ii) not to make the gas composition fall within the range of explosive composition. However, it is not easy to eliminate ignition sources, and the ignition may possibly occur, for example, by static electricity, and therefore, it is not easy to take perfect measures. Therefore, the measures are usually taken for the latter, that is, the management of the gas composition is preferentially performed. Combustible gasses generally have a very low burning or explosive risk when used alone. For causing burning or explosion of a combustible gas, when the combustible gas is mixed with a combustion-supporting gas (combustion-supporting component) such as oxygen at a certain ratio, and conditions such as temperature (or an ignition source) are satisfied, burning explosion is caused.

In the present description, the "combustion-supporting gas" refers to a gas that has a property of not burning alone, but promoting burning by being mixed with the combustible gas. Examples of the combustion-supporting gas include gasses such as oxygen, ozone, nitrous oxide, nitrogen monoxide, nitrogen dioxide, fluorine, chlorine, chlorine dioxide, nitrogen trifluoride, chlorine trifluoride, silicon tetrachloride, and oxygen difluoride. As the combustion-supporting gas applicable in the culture system 1 according to the present invention (and the method according to the present invention), the gasses described above are applicable. However, in consideration of the use for culture of microorganisms, the combustion-supporting gas preferably contains an oxygen gas or is an oxygen gas. As the combustion-supporting gas applicable in the culture system 1 according to the present invention (and the method according to the present invention), air containing an oxygen gas is also applicable in some cases. When a recycled gas is adjusted by adding air to the off-gas, the concentration of another gas component (for example, a nitrogen gas) contained in the air may be adjusted. In the present description, a "single combustion-supporting gas" does not mean a combustion-supporting gas containing no component other than the combustion-supporting gas, but means a combustion-supporting gas that can contain, for example, less than 5%, preferably less than 4%, 3%, 2%, 1%, 0.5%, or 0.1% of a component other than the combustion-supporting gas.

In the cases of a mixed system of two components, i.e., the combustible gas and the combustion-supporting gas, by setting the concentration of the combustible gas to the lower explosion limit or lower, or the upper explosion limit or higher of the mixed system, burning explosion can be prevented. The lower explosion limit means the lowest concentration of the combustible gas that enables continuation of burning, and the upper explosion limit means the lowest concentration of the combustion-supporting gas that enables continuation of burning. Accordingly, when the composition of the upper explosion limit is represented by the concentration of the combustion-supporting gas, explosion can be prevented by keeping the concentration of the combustion-supporting gas in the mixed system at the represented concentration or lower. When the combustion-supporting gas is an oxygen gas, the oxygen concentration of the upper explosion limit is particularly referred to as "explosion limit oxygen concentration" (see YAGYU Shozo, "Explosion limit oxygen concentration in addition of inert gas", Journal of Japan Society for Safety Engineering, Vol. 25, No. 4 (1986))

Accordingly, in the culture system 1 according to the present invention (and the method according to the present invention), by adjusting the amount of a gas component added by the recycled gas adjusting unit so that the concentration of the combustion-supporting gas in the recycled gas falls within the prescribed range, that is, lower than the explosion limit concentration (for example, lower than the explosion limit oxygen concentration), explosion of the recycled gas can be prevented, and safe and efficient fermentation culture can be performed. The explosion limit concentration varies according to a gas component that can be contained in the recycled gas, and therefore, it is possible to set as appropriate the explosion limit concentration on the basis of the known information according to the purpose of fermentation culture or the means (for example, the type of the microorganism, the type of the combustible gas, and the type of the incombustible gas) applied. (See, for example, YAGYU Shozo, "Explosion limit oxygen concentration in addition of inert gas", Journal of Japan Society for Safety Engineering, Vol. 25, No. 4 (1986).)

When the off-gas or the recycled gas contains at least three components including the incombustible gas as well as the combustible gas and the combustion-supporting gas, the mixing ratio between the three components may be set so as not to be included in the range of explosion limit on the basis of known information (see, example, YAGYU Shozo, "Explosion limit oxygen concentration in addition of inert gas", Journal of Japan Society for Safety Engineering, Vol. 25, No. 4 (1986); and YAGYU Shozo, "Explosion range of mixed gas (2)", Journal of Japan Society for Safety Engineering, Vol. 1, No. 2 (1962).). When the gas components contained in the off-gas or the recycled gas include, but are not limited to, for example, a component containing a hydrogen gas (combustible gas), a carbon dioxide gas (incombustible gas), and an oxygen gas (combustion-supporting gas), the upper explosion limit of the concentration of the oxygen gas is about 5%, being hardly affected by the ratio of the other two components, under the conditions of ambient temperature and atmospheric pressure. When the concentration of the oxygen gas needs to be increased, the concentration of the hydrogen gas can be set to 8% or less to be outside the explosion range. When the concentration of the carbon dioxide gas is 80%, the concentration of each of the hydrogen gas and the carbon dioxide gas can be tolerated up to 10%. Accordingly, the mixing ratio between the gas components may be adjusted according to, for example, the type of the microorganism applied in the present invention and the growth state thereof, and the type of the combustible gas and the incombustible gas contained in the off-gas or the recycled gas. At least from the viewpoint of safety, the concentration of the combustion-supporting gas (for example, the oxygen gas) may be controlled so as to fall within the prescribed range, and is preferably adjusted to lower than the explosion limit oxygen concentration. For example, when the combustible gas is a hydrogen gas, the oxygen concentration is preferably adjusted to less than 5%. The concentrations of the other gas components in the recycled gas are preferably adjusted, so as to respectively fall within prescribed ranges, according to the type of the microorganism used and for enabling efficient production of a desired substance by the microorganism.

In the culture system 1 according to the present invention (the method according to the present invention), for example, a helium gas, a nitrogen gas, water vapor, a carbon dioxide gas, or a carbon tetrachloride gas may be used as the incombustible gas, and these incombustible gasses may be used in combination of a plurality thereof. Since the explosion range varies by changing the incombustible gas, the incombustible gas can be selected as appropriate according to the purpose. In the culture system 1 according to the present invention (and the method according to the present invention), a carbon dioxide gas and/or a nitrogen gas may be contained as the incombustible gas. The incombustible gas used in the present invention may have a role as a substrate used for fermentation of the microorganism, the incombustible gas may be selected as appropriate according to the type of the microorganism, and, for example, a carbon dioxide gas may be used as a fermentation substrate of the microorganism.

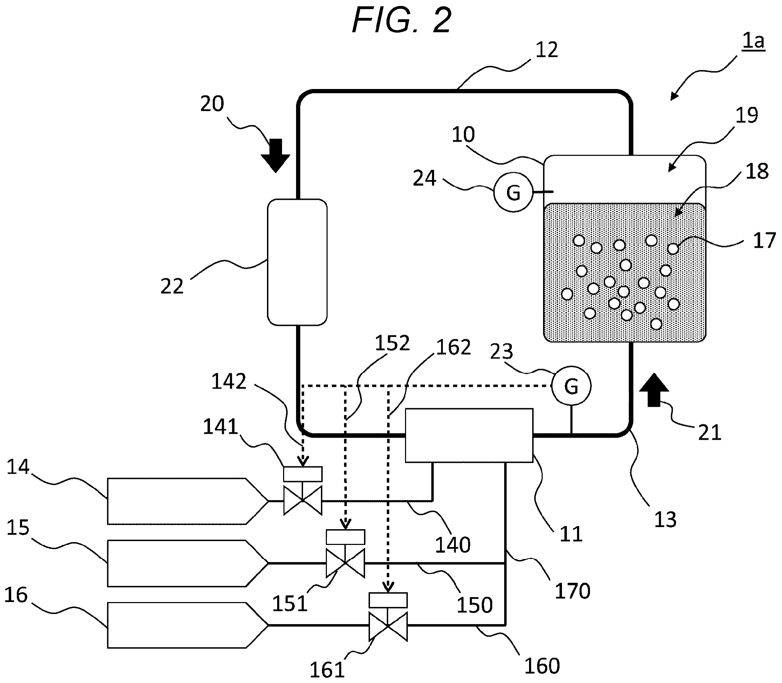

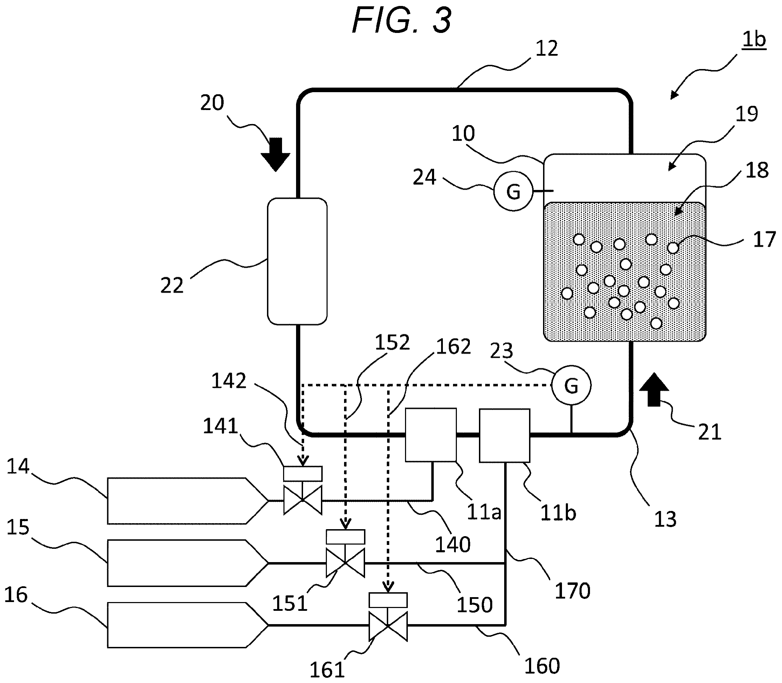

In one aspect (see, for example, FIGs. 2 and 3), the culture system may further include, in the second gas circulation line 13, a first gas concentration meter 23 for measuring at least the concentration of the combustion-supporting gas in the recycled gas. The recycled gas adjusting unit performs feedback adjustment, on the basis of the concentration of the combustion-supporting gas measured by the first gas concentration meter 23, so that the concentration of the combustion-supporting gas in the recycled gas falls within the prescribed range (for example, lower than the explosion limit concentration). Thereby, the concentration of the combustion-supporting gas in the recycled gas introduced into the culture tank can be controlled so as not to exceed the prescribed concentration, the concentration of the combustion-supporting gas can be set so as not to exceed the prescribed concentration in the whole culture system (1a, 1b) according to the present invention, and the safety can further be increased.

In one aspect, the combustible gas feeder 14 can be independently connected to the recycled gas adjusting unit. The combustible gas feeder 14 includes a single combustible gas, and does not substantially include another component such as the combustion-supporting gas, and therefore, the composition of the combustible gas included in the combustible gas feeder is not included in the range of explosive composition. Further, since the combustible gas feeder 14 is independently connected to the recycled gas adjusting unit, the concentration of the combustion-supporting gas is kept lower than the explosion limit concentration and the safety is therefore secured also in the line connecting the combustible gas feeder 14 to the recycled gas adjusting unit. In the present description, the "single combustible gas" does not mean a combustible gas containing no component other than the combustible gas, but means a combustible gas that can contain, for example, less than 5%, preferably less than 4%, 3%, 2%, 1%, 0.5%, or 0.1% of a component other than the combustible gas.

In one aspect, the recycled gas adjusting unit can be configured to mix the combustible gas and/or the incombustible gas so that a concentration of the gas component(s) in the recycled gas is in a prescribed range. The concentration(s) of the combustible gas and/or the incombustible gas is preferably adjusted, so as to respectively fall within the prescribed range(s), according to the type of the microorganism used and for enabling production of a desired substance by the microorganism. Thereby, the concentration of the component(s) other than the combustion-supporting gas in the recycled gas is also adjusted, and therefore, a recycled gas having conditions more suitable for fermentation of microorganisms can be supplied to the culture tank, enabling acceleration of efficient fermentation.

In one aspect (for example, FIGs. 2 and 3), the culture system may further include, in the upper part of the culture tank 10 and/or the first gas circulation line 12, a second gas concentration meter 24 for measuring at least the concentration of the combustion-supporting gas in the off-gas. Thereby, for example, even when the culture state of microorganisms is rapidly changed and the concentration of the combustion-supporting gas (for example, the oxygen gas) contained in the off-gas is deviated from the concentration range expected for normal culture (for example, when the concentration of oxygen in the off-gas does not become lower than the concentration range expected for normal culture), an abnormality can be detected in advance. On the basis of the abnormality detected, for example, the amount of the gas component(s) added in the recycled gas adjusting unit can be adjusted, the concentration of the combustion-supporting gas is prevented from falling outside the prescribed range, and the safety can be expected to be further increased. Accordingly, in one aspect, the culture system (1a, 1b) may also have a mechanism in which the recycled gas adjusting unit performs feedforward adjustment, on the basis of the concentration of the combustion-supporting gas measured by the second gas concentration meter 24, so that the concentration of the combustion-supporting gas in the recycled gas falls within the prescribed range.

The first gas concentration meter 23 and/or the second gas concentration meter 24 may further have a function of enabling measurement of the concentration of the combustible gas and/or the incombustible gas. Thereby, it is possible to confirm that the concentration of the combustible gas and/or the incombustible gas contained in the off-gas and/or the recycled gas is in the prescribed range, or particularly in the culture conditions suitable for culture of microorganisms.

In one aspect (FIGs. 2 and 3), the combustion-supporting gas feeder 15 and the incombustible gas feeder 16 may be connected to the recycled gas adjusting unit such that the combustion-supporting gas and the incombustible gas are premixed before mixed in the off-gas. For example, a combustion-supporting gas feed line 150 connected to the combustion-supporting gas feeder 15 and an incombustible gas feed line 160 connected to the incombustible gas feeder 16 may be joined together and thus form a combustion-supporting gas/incombustible gas feed line 170, and the combustion-supporting gas/incombustible gas feed line 170 may be connected to the recycled gas adjusting unit (for example, a gas mixer 11, 11b). Thereby, the combustion-supporting gas and the incombustible gas are premixed before mixed in the off-gas. By the premixing of the combustion-supporting gas and the incombustible gas, it is possible to lower the concentration of the combustion-supporting gas provided at high concentrations, reduce the burning risk due to the combustion-supporting gas being in a burnable range when mixed with the off-gas, and lower the risk of burning, corrosion, or the like of a metal material caused by the combustion-supporting gas.

The microorganism that can be cultured in the culture system 1 according to the present invention (and the method according to the present invention) is not particularly limited, but may be, for example, an aerobic fermentation microorganism or an anaerobic fermentation microorganism. As the aerobic fermentation microorganism, for example, a hydrogen-oxidizing bacterium can be used.

The hydrogen-oxidizing bacterium is a collective term for bacteria that oxidize free hydrogen and are assimilated into carbon dioxide, using the energy generated by the reaction.

The hydrogen-oxidizing bacterium may be, but not limited to, for example, a bacterium such as Achromobacter, Acidithiobacillus, Acidovorax, Alcaligenes, Anabena, Aquifex, Arthrobacter, Azospirillum, Bacillus, Bradyrhizobium, Cupriavidus, Derxia, Helicobacter, Herbaspirillum, Hydrogenobacter, Hydrogenobaculum, Hydrogenophaga, Hydrogenophilus, Hydrogenothermus, Hydrogenovibrio, Ideonella sp. O1, Kyrpidia, Metallosphaera, Methanobrevibacter, Myobacterium, Nocardia, Oligotropha, Paracoccus, Pelomonas, Polaromonas, Pseudomonas, Pseudonocardia, Rhizobium, Rhodococcus, Rhodopseudomonas, Rhodospirillum, Streptomyces, Thiocapsa, Treponema, Variovorax, Xanthobacter, and Wautersia. A plurality thereof may be used in combination.

In the culture system 1 according to the present invention (and the method according to the present invention) of one aspect, a pressure in the culture tank 10 can be adjusted to an atmospheric pressure or higher by any pressurizing means (for example, a pump or a compressor). Thereby, the recycled gas supplied to the culture tank 10 can efficiently be dissolved in the medium, and the fermentation culture of microorganisms can be promoted. The pressure in the culture tank 10 may be, for example, by gauge pressure (differential pressure against atmospheric pressure), for example, 0.01 MPa to 1.0 MPa, 0.01 MPa to 0.5 MPa, 0.01 MPa to 0.2 MPa, or 0.05 MPa to 0.2 MPa.

The culture system 1 according to one aspect can include a compressor in the first gas circulation line to circulate the off-gas. The compressor that can be applied in the present invention is, but not limited to, for example, an axial compressor, a centrifugal compressor, a reciprocating compressor, or a rotary compressor, and the compressor may be selected as appropriate in consideration of, for example, the purpose, the scale of the system, the gas composition of the off-gas and/or the recycled gas.

In the culture system 1 according to one aspect (FIGs. 1 to 3), the recycled gas adjusting unit can be, for example, one or a plurality of gas mixers (11, 11a, 11b). By the operation of the gas mixer(s) (11, 11a, 11b), the combustible gas, the combustion-supporting gas, and/or the incombustible gas supplied from the combustible gas feeder 14, the combustion-supporting gas feeder 15, and/or the incombustible gas feeder 16 is mixed with the off-gas, and the concentration of the combustion-supporting gas in the recycled gas is adjusted so as to fall within the prescribed range (for example, lower than the explosion limit oxygen concentration). To the single gas mixer (11), the combustible gas feeder 14 may be connected via a combustible gas feed line 140, the combustion-supporting gas feeder 15 may be connected via the combustion-supporting gas feed line 150, and the incombustible gas feeder 16 may be connected via the incombustible gas feed line 160. Alternatively, to the plurality of gas mixers (11a, 11b), the combustible gas feeder 14, the combustion-supporting gas feeder 15, and the incombustible gas feeder 16 may respectively be connected. For example, the combustible gas feeder 14, the combustion-supporting gas feeder 15, and the incombustible gas feeder 16 may respectively be connected to three gas mixers. Alternatively, for example, the combustible gas feeder 14 may be connected to one gas mixer 11a of two gas mixers (11a, 11b), and the combustion-supporting gas feeder 15 and the incombustible gas feeder 16 may be connected to the other gas mixer 11b.

In the culture system (1a, 1b) according to one aspect (for example, FIGs. 2 and 3), the recycled gas adjusting unit may include:
one or a plurality of gas mixers (11, 11a, 11b);
a first gas adjusting unit 141 for adjusting an amount of the combustible gas supplied from the combustible gas feeder 14;
a second gas adjusting unit 151 for adjusting an amount of the combustion-supporting gas supplied from the combustion-supporting gas feeder 15; and
a third gas adjusting unit 161 for adjusting an amount of the incombustible gas supplied from the incombustible gas feeder 16.

The first gas adjusting unit 141, the second gas adjusting unit 151, and/or the third gas adjusting unit 161 may be disposed respectively halfway through the combustible gas feed line 140, the combustion-supporting gas feed line 150, and/or the incombustible gas feed line 160 (for example, FIGs. 2 and 3), and the combustible gas feeder 14, the combustion-supporting gas feeder 15, and/or the incombustible gas feeder 16 may be disposed at a gas outlet. The first gas adjusting unit 141, the second gas adjusting unit 151, and/or the third gas adjusting unit 161 can adjust the amount of the supplied gas component by a closing mechanism such as a valve (for example, an electromagnetic valve). The first gas adjusting unit 141, the second gas adjusting unit 151, and/or the third gas adjusting unit 161 undergoes as appropriate feedback adjustment according to the gas composition of the recycled gas measured by the first gas concentration meter 23, and this feedback adjustment prevents the gas composition of the recycled gas from being included in the explosion range and enables the safety to be further increased.

### <Method for culturing microorganism, with off-gas recycled, in culture system using microorganism>

In one aspect, a method according to the present invention can include:
a step (1) of culturing a microorganism by supplying a supply gas containing a combustible gas, a combustion-supporting gas, and an incombustible gas to a culture material that is provided in a culture tank of a culture system and includes the microorganism and a medium;
a step (2) of preparing a recycled gas by mixing, in at least a portion of an off-gas discharged by the step (1), one or a plurality of gas components selected from the group consisting of the combustible gas, the combustion-supporting gas, and the incombustible gas, the mixing being performed so that a concentration of the combustion-supporting gas in the recycled gas is in a prescribed range; and
a step (3) of performing the step (1) by supplying, as the supply gas, the recycled gas obtained in the step (2) to the culture material. The method according to the present invention can be performed, for example, by employing the culture system described above, but the configuration of the culture system is not limited only to the one described above as long as the method according to the present invention can be performed. As the configuration applied in the method according to the present invention, the structural elements described in the <System for culturing microorganism> described above can be applied.

In the method according to one aspect, the medium used in the system and the method according to the present invention is selected as appropriate according to the type of the microorganism used, and is not particularly limited because a known medium or a medium having the composition thereof changed as appropriate according to the purpose is used.

In one aspect, in the step (2), a recycled gas is prepared by mixing, in at least a portion of an off-gas discharged by the step (1), one or a plurality of gas components selected from the group consisting of the combustible gas, the combustion-supporting gas, and the incombustible gas. As described above, the concentration of the combustion-supporting gas in the recycled gas may be controlled so as to fall within the prescribed range, and is more preferably set to lower than the explosion limit concentration in terms of safety. The concentrations of the other gas components in the recycled gas are preferably adjusted, so as to respectively fall within prescribed ranges, according to the type of the microorganism used and for enabling efficient production of a desired substance by the microorganism.

In one aspect, the method according to the present invention may include, after the step (2) but before the step (3),
a step (2') of measuring at least the concentration of the combustion-supporting gas in the recycled gas, wherein feedback adjustment is performed, on the basis of the concentration of the combustion-supporting gas measured in the step (2'), so that the concentration of the combustion-supporting gas in the step (2) falls within the prescribed range (for example, lower than the explosion limit concentration). Thereby, the concentration of the combustion-supporting gas in the recycled gas introduced into the culture tank can be controlled so as not to exceed the prescribed concentration, the concentration of the combustion-supporting gas can be set so as not to exceed the prescribed concentration in the whole system having the method according to the present invention applied thereto, and the safety can further be increased.

In one aspect, the combustible gas mixed in the method according to the present invention is preferably independently mixed in the off-gas. By independently supplying a single combustible gas to the off-gas, the concentration of the combustion-supporting gas in the combustible gas is kept lower than the explosion limit concentration until the combustible gas is mixed with the off-gas, and therefore, the safety is secured.

In one aspect, the combustible gas and/or the incombustible gas mixed in the method according to the present invention is mixed in the gas components so as to be in a prescribed range in the recycled gas. The concentration(s) of the combustible gas and/or the incombustible gas is preferably adjusted, so as to respectively fall within the prescribed range(s), according to the type of the microorganism used and for enabling production of a desired substance by the microorganism. Thereby, the concentration of the component(s) other than the combustion-supporting gas in the recycled gas is also adjusted, and therefore, a gas having conditions more suitable for fermentation can be supplied to the culture tank, enabling acceleration of efficient fermentation.

The method according to the present invention of one aspect may include, after the step (1) but before the step (2),
a step (1') of measuring at least the concentration of the combustion-supporting gas in the off-gas discharged by the step (1). Thereby, for example, even when the culture state of microorganisms is rapidly changed and the concentration of oxygen contained in the off-gas is rapidly changed (for example, when the oxygen concentration is not lowered), such a change can be detected before generation of the recycled gas, the concentration of the combustion-supporting gas in the recycled gas can be prevented from falling outside the prescribed range, and the safety can be expected to be further increased.

Accordingly, the method according to the present invention of one aspect may include, after the step (1) but before the step (2),
a step (1'') of measuring at least the concentration of the combustion-supporting gas in the off-gas discharged by the step (1), wherein feedforward adjustment is performed, on the basis of the concentration of the combustion-supporting gas measured in the step (1''), so that the concentration of the combustion-supporting gas in the step (2) falls within the prescribed range.

Each of the steps of the present invention in one aspect may include measuring a concentration of the combustible gas and/or the incombustible gas contained in the off-gas and/the recycled gas, and adjusting the concentration of the combustible gas and/or the incombustible gas to the prescribed range, particularly to the culture conditions suitable for culture of microorganisms.

In the step (2) of the method according to the present invention of one aspect, the combustion-supporting gas and the incombustible gas may be premixed before mixed in the off-gas. Thereby, it is possible to lower the concentration of the combustion-supporting gas provided at high concentrations, reduce the burning risk due to the combustion-supporting gas being in a burnable range when mixed with the off-gas, and lower the risk of burning, corrosion, or the like of a metal material caused by the combustion-supporting gas.

### Reference Signs List

- 1, 1a, 1b: Culture system
- 10: Culture tank
- 11, 11a, 11b: Gas mixer
- 12: First gas circulation line
- 13: Second gas circulation line
- 14: Combustible gas feeder
- 15: Combustion-supporting gas feeder
- 16: Incombustible gas feeder
- 17: Microorganism
- 18: Medium
- 19: Off-gas
- 20: Flowing direction of off-gas
- 21: Flowing direction of recycled gas
- 22: Compressor
- 23: First gas concentration meter
- 24: Second concentration meter
- 140: Combustible gas feed line
- 141: First gas adjusting unit
- 142: Feedback control signal sent to first gas adjusting unit
- 150: Combustion-supporting gas feed line
- 151: Second gas adjusting unit
- 152: Feedback control signal sent to second gas adjusting unit
- 160: Incombustible gas feed line
- 161: Third gas adjusting unit
- 162: Feedback control signal sent to third gas adjusting unit
- 170: Combustion-supporting gas/incombustible gas feed line

## Claims

1. A system for culturing a microorganism, the system comprising:
a culture tank;
a recycled gas adjusting unit;
a first gas circulation line having one end thereof connected to an upper part of the culture tank and another end thereof connected to the recycled gas adjusting unit; and
a second gas circulation line having one end thereof connected to the recycled gas adjusting unit and another end thereof connected so as to supply a recycled gas into the culture tank,
the recycled gas adjusting unit being connected to one or a plurality of gas feeders selected from the group consisting of a combustible gas feeder, a combustion-supporting gas feeder, and an incombustible gas feeder, and the recycled gas adjusting unit mixing, in at least a portion of an off-gas discharged from the culture tank, one or a plurality of gas components selected from the group consisting of a combustible gas, a combustion-supporting gas, and an incombustible gas so that a concentration of the combustion-supporting gas in the recycled gas is in a prescribed range.

2. The system according to claim 1, further comprising, in the second gas circulation line, a first gas concentration meter for measuring at least the concentration of the combustion-supporting gas in the recycled gas, wherein
the recycled gas adjusting unit performs feedback adjustment, on the basis of the concentration of the combustion-supporting gas measured by the first gas concentration meter, so that the concentration of the combustion-supporting gas in the recycled gas falls within the prescribed range.

3. The system according to claim 1 or 2, wherein the combustible gas feeder is independently connected to the recycled gas adjusting unit.

4. The system according to any one of claims 1 to 3, wherein the recycled gas adjusting unit mixes the gas components so that a concentration of the combustible gas and/or the incombustible gas in the recycled gas is in a prescribed range.

5. The system according to any one of claims 1 to 4, further comprising, in the upper part of the culture tank and/or the first gas circulation line, a second gas concentration meter for measuring at least the concentration of the combustion-supporting gas in the off-gas.

6. The system according to any one of claims 1 to 5, wherein the combustion-supporting gas feeder and the incombustible gas feeder are connected to the recycled gas adjusting unit such that the combustion-supporting gas and the incombustible gas are premixed before mixed in the off-gas.

7. The system according to any one of claims 1 to 6, wherein the microorganism is an aerobic fermentation microorganism.

8. The system according to claim 7, wherein the aerobic fermentation microorganism is a hydrogen-oxidizing bacterium, and the combustible gas is a hydrogen gas.

9. The system according to any one of claims 1 to 8, wherein the combustion-supporting gas is an oxygen gas.

10. The system according to any one of claims 1 to 9, wherein the incombustible gas is a carbon dioxide gas and/or a nitrogen gas.

11. The system according to any one of claims 1 to 10, wherein a pressure in the culture tank is an atmospheric pressure or higher.

12. The system according to any one of claims 1 to 11, comprising a compressor in the first gas circulation line.

13. The system according to any one of claims 1 to 12, wherein the recycled gas adjusting unit includes:
one or a plurality of gas mixers;
a first gas adjusting unit for adjusting an amount of the combustible gas supplied from the combustible gas feeder;
a second gas adjusting unit for adjusting an amount of the combustion-supporting gas supplied from the combustion-supporting gas feeder; and
a third gas adjusting unit for adjusting an amount of the incombustible gas supplied from the incombustible gas feeder, and
the combustible gas feeder, the combustion-supporting gas feeder, and the incombustible gas feeder are connected to the gas mixers.

14. A method for culturing a microorganism, with an off-gas recycled, in a culture system using a microorganism, the method comprising:
a step (1) of culturing a microorganism by supplying a supply gas containing a combustible gas, a combustion-supporting gas, and an incombustible gas to a culture material that is provided in a culture tank of a culture system and includes the microorganism and a medium;
a step (2) of preparing a recycled gas by mixing, in at least a portion of an off-gas discharged by the step (1), one or a plurality of gas components selected from the group consisting of the combustible gas, the combustion-supporting gas, and the incombustible gas, the mixing being performed so that a concentration of the combustion-supporting gas in the recycled gas is in a prescribed range; and
a step (3) of performing the step (1) by supplying, as the supply gas, the recycled gas obtained in the step (2) to the culture material.

15. The method according to claim 14, the method comprising, after the step (2) but before the step (3),
a step (2') of measuring at least the concentration of the combustion-supporting gas in the recycled gas, wherein feedback adjustment is performed, on the basis of the concentration of the combustion-supporting gas measured in the step (2'), so that the concentration of the combustion-supporting gas in the step (2) falls within the prescribed range.

16. The method according to claim 14 or 15, wherein the combustible gas is independently mixed in the at least a portion of the off-gas.

17. The method according to any one of claims 14 to 16, wherein the combustible gas and/or the incombustible gas is mixed in the gas components so as to be in a prescribed concentration range in the recycled gas.

18. The method according to any one of claims 14 to 17, comprising, after the step (1) but before the step (2),
a step (1') of measuring at least the concentration of the combustion-supporting gas in the off-gas discharged by the step (1).

19. The method according to any one of claims 14 to 18, wherein in the step (2), the combustion-supporting gas and the incombustible gas are premixed before mixed in the at least a portion of the off-gas.

20. The method according to any one of claims 14 to 19, wherein the microorganism is an aerobic fermentation microorganism.

21. The method according to claim 20, wherein the aerobic fermentation microorganism is a hydrogen-oxidizing bacterium, and the combustible gas is a hydrogen gas.

22. The method according to any one of claims 14 to 21, wherein the combustion-supporting gas is an oxygen gas.

23. The method according to any one of claims 14 to 22, wherein the incombustible gas is a carbon dioxide gas and/or a nitrogen gas.

24. The method according to any one of claims 14 to 23, wherein a pressure in the culture tank is an atmospheric pressure or higher.
